# FASCICULE DE BREVET EUROPEEN

(11) **EP 2 111 460 B1**
(45) Date de publication et mention de la délivrance du brevet: **11.12.2019**
(21) Numéro de dépôt: 08762041.5
(22) Date de dépôt: 07.02.2008
(51) Int. Cl.: C12Q 1/04, C12N 1/20, C12Q 1/10, G01N 33/569

(54) **MILIEU DE DÉTECTION ET/OU D'IDENTIFICATION DE BACTÉRIES**
MEDIUM FÜR DEN NACHWEIS UND/ODER DIE IDENTIFIZIERUNG VON BAKTERIEN
MEDIUM FOR THE DETECTION AND/OR IDENTIFICATION OF BACTERIA

(30) Priorité: 08.02.2007 FR 0753149
(43) Date de publication de la demande: 28.10.2009
(73) Titulaire: BIOMERIEUX, 69280 Marcy-L'Etoile (FR)
(72) Inventeur: CASSE, Marine, F-38380 Saint Pierre de Chartreuse (FR); ORENGA, Sylvain, F-01160 Neuville Sur Ain (FR); ROGER-DALBERT, Céline, G1X4T8 Québec (CA)
(74) Mandataire: Sprugnoli, Claude Louis Victor
(86) Numéro de dépôt international: PCT/FR2008/050183
(87) Numéro de publication internationale: WO 2008/104679

(56) Documents cités:
- EP-A- 0 704 538
- EP-A- 1 300 471
- EP-A1- 1 323 832
- WO-A1-95/30024
- US-A- 5 871 944
- KANTHAM L ET AL: "BETA GLUCOSIDASE OF PENICILLIUM-FUNICULOSUM 2. PROPERTIES AND MYCELIAL BINDING" BIOTECHNOLOGY AND BIOENGINEERING, vol. 27, no. 6, 1985, pages 786-791, XP002491178 ISSN: 0006-3592
- MARTIN C ET AL: "INTERET DES MILIEUX CONTENANT DES SUBSTRATS CHROMOGENES POUR L'IDENTIFICATION ET LA NUMERATION DES BACTERIES URINAIRES" PATHOLOGIE ET BIOLOGIE, L'EXPANSION SCIENTIFIQUE FRANCAISE, PARIS, FR, vol. 43, no. 9, novembre 1995 (1995-11), pages 749-753, XP002047436 ISSN: 0369-8114
- CIRAGIL P ET AL: "Evaluation of a new chromogenic medium for isolation and identification of common urinary tract pathogens", EUROPEAN JOURNAL OF CLINICAL MICROBIOLOGY & INFECTIOUS DISEASES, SPRINGER, BERLIN, DE, vol. 25, no. 2, 1 February 2006 (2006-02-01), pages 108-111, XP019355471, ISSN: 1435-4373, DOI: 10.1007/S10096-006-0103-5

## Description

Le domaine de l'invention est celui de l'analyse microbiologique par voie biochimique, et en particulier de la détection et de l'identification de bactéries.

Les bactéries pathogènes, et notamment les bacilles à Gram négatif, tels que les entérobactéries, les *Vibrionaceae*, les *Pseudomonas*, sont responsables chaque année de nombreuses maladies, épidémies...

Les *Pseudomonas* sont des bactéries ubiquitaires que l'on rencontre dans les sols, sur les végétaux et surtout dans les eaux douces et marines. De nombreuses souches peuvent se développer à basse température, et contaminent de ce fait les aliments conservés au réfrigérateur, et sont à l'origine d'infections digestives. Elles sont également à l'origine d'infections nosocomiales.

Les espèces d'entérobactéries les plus communément isolées en bactériologie clinique appartiennent aux genres *Citrobacter*, *Enterobacter*, *Escherichia*, *Hafnia*, *Klebsiella*, *Morganella*, *Proteus, Providencia, Salmonella, Serratia*, *Shigella, Yersinia.*

L'espèce *E. coli* est l'espèce aérobie la plus représentée dans le tube digestif. Toutefois, leur présence dans l'eau est un témoin de contamination fécale, et certaines souches sont pathogènes et responsables de suppurations péritonéales, biliaires, appendiculaires ou génitales.

Les bactéries du genre *Salmonella* sont des parasites intestinaux des animaux vertébrés et des oiseaux et sont transmises à l'homme par le biais d'aliments contaminés. Elles sont alors responsables de maladies gastro-entérites et fièvres typhoïde et paratyphoïdes. Enfin, les *Citrobacter,* tel que *Citrobacter freundii*, sont des commensaux du tube digestif de l'homme et des animaux qu'on peut isoler des urines, des sécrétions respiratoires voire du sang, et qui sont responsables d'infections chez les sujets immunodéprimés.

Une détection précoce et spécifique de ces bactéries à Gram négatif permet de proposer une solution adaptée, en terme de traitement, de décontamination...

Il existe actuellement de nombreux milieux permettant la détection des bactéries à Gram négatif. Cette détection peut être basée notamment sur l'utilisation de substrats particuliers, spécifiques d'une activité métabolique, telle qu'une activité enzymatique, de la bactérie que l'on souhaite détecter : par le choix des substrats, selon qu'il y a réaction ou non, il est possible de caractériser la nature d'un microorganisme.

On peut citer notamment le milieu UriSelect 4 (Bio-Rad), qui utilise un substrat de β-galactosidase associé à un substrat de β-glucosidase et à la détection de la tryptophanase pour la détection des souches de l'espèce *E. coli* et la séparation des souches de *Citrobacter* β-galactosidase positives / β-glucosidase négatives.

On peut citer également le milieu UTI (Oxoid) qui utilise un substrat de β-galactosidase associé à un substrat de β-glucosidase et à la détection de la tryptophanase pour la détection des souches de l'espèce *E. coli* et la séparation des souches de *Citrobacter* β-galactosidase positives / β-glucosidase négatives.

Le milieu CPS ID 3 (bioMerieux) utilise un substrat de β-glucuronidase associé à un substrat de β-glucosidase et éventuellement à la détection de la tryptophanase pour la détection des souches de l'espèce *Escherichia coli.* Ciragil et al., 2006 évalue les performance de ce milieu chromogénique pour l'isolement et l'identification des pathogènes du tractus urinaire (European Journal of Clinical Microbiology and Infectious Diseases; 2006 (25): 108-111).

Le milieu BBL CHROMagar Orientation (Becton-Dickinson) utilise un substrat de β-galactosidase associé à un substrat de β-glucosidase et à la détection de la tryptophanase pour la détection des souches de l'espèce *E. coli* et la séparation des souches de *Citrobacter* β-galactosidase positives / β-glucosidase négatives.

Enfin, le milieu Urine Specific Agar (AES Laboratoire) utilise un substrat de β-glucuronidase associé à la détection de la tryptophanase pour la détection des souches de l'espèce *E. coli* et leur différenciation des souches de *Citrobacter.*

Toutefois, les milieux chromogènes utilisant un substrat de β-glucuronidase pour la détection des *E. coli* présentent une excellente spécificité, mais une sensibilité imparfaite du fait de l'existence d'une faible proportion de souches d'*E*. *coli* (5-10%) n'exprimant pas cette activité. De plus, certaines souches de *Citrobacter* peuvent également produire des colonies β-glucuronidase positives, de la même couleur que celles d'*E*. *coli*

Pour les milieux chromogènes utilisant un substrat de β-galactosidase pour la détection des *E. coli,* il est nécessaire de confirmer l'identification en effectuant un test supplémentaire pour différencier *E. coli* des autres entérobactéries exprimant une activité β-galactosidase, et n'exprimant pas ou faiblement une activité β-glucosidase, notamment certaines souches de *Citrobacter.*

En ce qui concerne la détection des salmonelles, on peut citer le milieu SM ID qui utilise du glucuronate et un substrat de β-galactosidase. Toutefois, une confirmation biochimique et/ou sérologique est nécessaire pour la détection des souches du genre *Salmonella* et leur différenciation des souches d'autres entérobactéries notamment celles du genre *Citrobacter.*

On peut citer enfin, en ce qui concerne les *Pseudomonas*, le milieu Cétrimide qui utilise du Cétrimide et la détection de la pyocyanine pour détecter les souches de l'espèces *Pseudomonas aeruginosa* et les différencier des autres bactéries à Gram négatif. Une amélioration de la spécificité de ce milieu serait toutefois un avantage.

L'invention se propose de résoudre les problèmes de l'état de la technique en présentant un nouveau milieu particulièrement adapté pour identifier les bactéries *Citrobacter* parmi d'autres entérobactéries, d'une manière rapide, peu coûteuse et aisée à mettre en œuvre.

D'une manière surprenante, les inventeurs ont montré qu'un milieu comprenant une combinaison particulière de substrats d'activité métabolique et d'inducteurs permettait une détection rapide et aisée des bactéries à Gram négatif. Plus précisément, les inventeurs ont notamment montré que l'induction de la β-glucosidase par un hydrate de carbone, en particulier le cellobiose permet de réduire le nombre de souches de *Citrobacter* produisant des colonies de même couleur que les *E. coli,* permettant ainsi une excellente séparation de ces deux espèces.

Avant d'aller plus avant dans l'exposé de l'invention, les définitions suivantes sont données afin de faciliter la compréhension de l'invention.

Par milieu de détection, on entend un milieu comprenant tous les éléments nécessaires à la survie et/ou à la croissance de microorganismes. Ce milieu de détection peut soit servir uniquement de milieu de détection, soit de milieu de culture et de détection. Dans le premier cas, la culture des microorganismes est effectuée avant ensemencement et, dans le deuxième cas, le milieu de détection constitue également le milieu de culture. Le milieu de culture selon l'invention peut contenir d'éventuels autres additifs comme par exemple : des peptones ou extraits de tissus, un ou plusieurs facteurs de croissance, des hydrates de carbone, un ou plusieurs agents sélectifs, des tampons, un ou plusieurs gélifiants... Ce milieu de culture peut se présenter sous forme de liquide, de gel prêt à l'emploi, c'est à dire prêt à l'ensemencement en tube, flacon, ou sur boite de Petri.

Au sens de la présente invention, la détection peut être réalisée en milieu liquide, bandelette ou autre support solide

Par groupe de bactéries à Gram négatif, on entend un groupe de bactéries qui, lors de la coloration par la méthode de Gram, éliminent le cristal violet et apparaissent rose.

Un tel groupe de bactéries à Gram négatif peut comprendre notamment, ou être constitué notamment d'entérobactéries, de salmonelles (*Salmonella*), de *Pseudomonas aeruginosa.* Comme bactéries à Gram négatif, on peut citer notamment la famille des entérobactéries (*Enterobacteriaceae*), la famille des *Vibrionaceae,* telle que l'espèce *Vibrio cholerae,* ou encore les *Pseudomonas,* en particulier *Pseudomonas aeruginosa.*

Par entérobactéries, on peut citer notamment les genres suivants *Alishewanella* ; *Alterococcus ; Aquimonas ; Aranicola ; Arsenophonus ; Azotivirga ; Blochmannia (candidatus) ; Brenneria ; Buchnera ; Budvicia ; Buttiauxella ; Calymmatobacterium ; Cedecea ; Citrobacter ; Dickeya ; Edwardsiella ; Enterobacter ; Erwinia,* par exemple *Erwinia amylovora ; Escherichia,* par exemple *Escherichia coli ; Ewingella ; Grimontella ; Hafnia ; Klebsiella,* par exemple *Klebsiella pneumoniae ; Kluyvera ; Leclercia ; Leminorella ; Levinea ; Moellerella ; Morganella ; Obesumbacterium ; Pantoea ; Pectobacterium ; Phlomobacter (candidatus) ; Photorhabdus ; Plesiomonas,* par exemple *Plesiomonas shigelloides ; Pragia ; Proteus,* par exemple *Proteus vulgaris ; Providencia ; Rahnella ; Raoultella ; Saccharobacter ; Salmonella ; Samsonia ; Serratia*, par exemple *Serratia marcescens ; Shigella ; Sodalis ; Tatumella ; Thorsellia, Trabulsiella ; Wigglesworthia ; Xenorhabdus ; Yersinia,* par exemple *Yersinia pestis* ; *Yokenella*

Par groupe comprenant *Citrobacter,* on entend un groupe comprenant des bactéries appartenant au genre *Citrobacter.* Ce groupe peut comprendre également d'autres espèces. Ainsi, un groupe comprenant *Citrobacter* préféré est un groupe comprenant, ou constitué de bactéries *Citrobacter, Enterobacter, Klebsiella,* et *Serratia* (appelé aussi groupe KESC).

Par substrat, on entend toute molécule susceptible d'engendrer directement ou indirectement un signal détectable dû à une activité enzymatique ou métabolique du microorganisme.

Le substrat peut être notamment un substrat métabolique, telle qu'une source de carbone ou d'azote, couplée à un indicateur produisant une coloration en présence de l'un des produits du métabolisme.

Le substrat peut être également un substrat enzymatique, c'est à dire un substrat pouvant être hydrolysé par une enzyme en un produit permettant la détection, directe ou indirecte d'un microorganisme. Ce substrat peut notamment comprendre une première partie spécifique de l'activité enzymatique à révéler et une seconde partie faisant office de marqueur, ci-après appelée partie marqueur. Cette partie marqueur peut être chromogène, fluorogène, luminescente... Comme substrat chromogène, bien adapté aux supports solides (filtre, gélose, gel d'électrophorèse), on peut citer notamment les substrats à base d'indoxyl et ses dérivés, et les substrats à base d'hydroxyquinoline ou d'esculétine et leurs dérivés, qui permettent la détection d'activités osidase et estérase. On peut citer également les substrats à base de nitrophénol et nitroaniline et dérivés, permettant de détecter les activités osidases et estérases dans le cas de substrats à base de nitrophénol, et des activités peptidases dans le cas de substrats à base de la nitroaniline. On peut citer enfin les substrats à base de naphtol et naphtylamine et leurs dérivés, qui permettent de détecter les activités osidases et estérases par l'intermédiaire du naphtol, et les activités peptidases par l'intermédiaire de la naphtylamine. Ce substrat peut permettre notamment, mais d'une façon non limitative, la détection d'une activité enzymatique telle que l'activité d'une osidase, peptidase, estérase... Le substrat enzymatique peut également être un substrat naturel dont le produit d'hydrolyse est détecté directement ou indirectement. Comme substrat naturel, on peut notamment citer le Tryptophane pour détecter une activité tryptophanase ou desaminase, un acide aminé cyclique (Tryptophane, Phénylalanine, Histidine, Tyrosine) pour détecter une activité désaminase, le Phosphatidyl Inositol pour détecter une activité phospholipase, ...

Selon la présente invention, le substrat est préférentiellement choisi parmi les substrats à base d'Indoxyl (3-Indoxyl, 5-Bromo-3-indoxyl, 4-Chloro-3-indoxyl, 5-Iodo-3-indoxyl, 5-Bromo-4-chloro-3-indoxyl, 5-Bromo-6-chloro-3-indoxyl, 6-Bromo-3-indoxyl, 6-Chloro-3-indoxyl, 6-Fluoro-3-indoxyl, 5-Bromo-4-chloro-N-méthyl-3-indoxyl, N-Méthyl-3-indoxyl, ...) ; d'umbelliférone (4-Méthylumbelliférone, Cyclohexenoesculétine, ...) ; d'Alizarine ; de p-Naphtolbenzéine ; de Nitrophénol (ortho-Nitrophénol, para-Nitrophénol, ...) ; de Naphtol (alpha-Naphtol, 2-Naphtol, Naphtol-ASBI, ...) ; d'Aminophénol (para-Aminophénol, Dichloro-aminophénol, ...) ; d'Hydroxyquinoline, ; de Cathécol (Cathécol, Dihydroxyflavone, Hydroxyflavone, ...),; de Résorufine ; de Chlorophénol Red ; de Fluorescéine ; d'Aminocoumarine (7-Amino-4-méthyl-coumarine, ...) ; de Naphtylamide ; d'Acridine (Amino-phényl-acridine) ; d'Amino-phénoxazine (Amino-benzophénoxazinone, Amino-pentyl-resorufine, ...).

Par activité métabolique, on entend un ensemble de réactions chimiques se produisant dans une bactérie. Ces réactions chimiques peuvent être liées à une ou plusieurs activités enzymatiques, à la dégradation, la synthèse, la modification d'une molécule.

Par activité métabolique spécifique d'un groupe de bactéries à Gram négatif, on entend une activité métabolique produite préférentiellement par ce groupe de bactéries à Gram négatif.

On peut citer notamment pour un groupe comprenant, ou constitué de, *E. coli,* la beta-glucuronidase, beta-galactosidase, alpha-galactosidase, acidification du Lactose, de tryptophanase, beta-ribosidase, phosphatase, L-Alanine-aminopeptidase, L-Leucine-aminopeptidase. Préférentiellement, l'activité métabolique est la beta-glucuronidase ou beta-galactosidase.

Les substrats utilisés pour la détection d'une activité beta-glucuronidase peuvent notamment être le 4-Méthylumbelliféryl-beta-glucuronide, le 5-Bromo-4-chloro-3-indolyl-beta-glucuronide, le 5-Bromo-6-chloro-3-indolyl-beta-glucuronide, le 6-Chloro-3-indolyl-beta-glucuronide, l'Alizarine-beta-glucuronide, le Cyclohexenoesculetine-beta-glucuronide ou leurs sels à des concentrations comprises préférentiellement entre 10 et 1000mg/l.

Les substrats utilisés pour la détection d'une activité beta-galactosidase peuvent notamment être le 4-Méthylumbelliféryl-beta-galactoside, le 5-Bromo-4-chloro-3-indolyl-beta-galactoside, le 5-Bromo-6-chloro-3-indolyl-beta-galactoside, le 6-Chloro-3-indolyl-beta-galactoside, l'Alizarine-beta-galactoside, le Cyclohexenoesculetine-beta-galactoside ou leurs sels à des concentrations comprises préférentiellement entre 10 et 1000mg/l.

On peut citer également pour un groupe comprenant, ou constitué de, *Salmonella,* l'alpha galactosidase, estérase, acidification du glucuronate, sorbitol, propanediol, mélibiose, mannitol. Comme substrat d'alpha-galactosidase, on peut citer le 4-Méthylumbelliféryl-alpha-galactoside, le 5-Bromo-4-chloro-3-indolyl-alpha-galactoside, le 5-Bromo-6-chloro-3-indolyl-alpha-galactoside, le 6-Chloro-3-indolyl-alpha-galactoside, l'Alizarine-alpha-galactoside, le Nitrophényl-alpha-galactoside à des concentrations comprises entre 10 et 1000mg/l/.

Parmi les substrats d'estérase, on peut citer le 4-Méthylumbelliféryl-octanoate, le 5-Bromo-4-chloro-3-indoxyl-octonaoate, 5-Bromo-4-chloro-3-indoxyl-nonaoate, le 5-Bromo-6-chloro-3-indoxyl-octanoate, 5-Bromo-6-chloro-3-indoxyl-hexanoate, le 6-Chloro-3-indoxyl-octanoate, l'Alizarine-octanoate, à des concentrations comprises entre 20 et 1000mg/l/.

On peut citer aussi pour un groupe comprenant, ou constitué de, *Pseudomonas aeruginosa,* l'estérase, l'aminopeptidase, l'oxydase.

Parmi les substrats d'estérase, on peut citer le 4-Méthylumbelliféryl-octanoate, le 5-Bromo-4-chloro-3-indoxyl-octonaoate, 5-Bromo-4-chloro-3-indoxyl-nonaoate, le 5-Bromo-6-chloro-3-indoxyl-octanoate, 5-Bromo-6-chloro-3-indoxyl-hexanoate, le 6-Chloro-3-indoxyl-octanoate, l'Alizarine-octanoate, à des concentrations comprises entre 20 et 1000mg/l/.

Parmi les substrats d'aminopeptidase, on peut notamment citer le beta-Alanyl-amidophénol, le beta-Alanyl-dichloro-amidophénol, beta-Alanyl-para-nitroanilide, beta-Alanyl-beta-naphtylamide, le 7-N-(beta-Alanyl) aminophenoxazine-1-pentyl-3-one, le 7-N-(L-Pyroglutamyl) aminophenoxazine-1-pentyl-3-one à des concentrations comprises entre 10 et 1000mg/l.

Par inducteur, on entend un composé induisant une augmentation de l'expression de l'activité métabolique ciblée, toute condition expérimentale étant égale par ailleurs, l'activité métabolique est plus forte lorsque l'inducteur est à une concentration appropriée que lorsqu'il est absent ou à une concentration inadaptée.

On peut citer notamment :
- pour la beta glucosidase ou la cellobiosidase, un hydrate de carbone constitué d'un hydrate de carbone lié en position β au glucose ou hydrate de carbone avec une sous-unité β-glucoside, en particulier le cellobiose, la cellulose, l'amidon, le cellotriose, le trehalose.
   On peut citer également le Méthyl-β-glucoside, l'Isopropyl-β-thio-glucoside, l'Indoxyl-β-glucoside ou le Méthyl-β-thio-glucoside.
- pour la beta-glucuronidase, le glucuronate, le Méthyl-beta-glucuronide ou autres beta-glucuronides.
- Pour la beta-galactosidase, le Lactose, l'Isopropyl-beta-thio-galactoside ou autres beta-galactoside.

Sans être limitatif, une concentration comprise entre 100ng/l et 10g/l, préférentiellement comprise entre 10mg/l et 3g/l est particulièrement adaptée a la présente invention.

Par échantillon biologique, on entend un échantillon clinique, issu d'un prélèvement de liquide biologique, ou un échantillon alimentaire, issu de tout type d'aliment ou un échantillon d'environnement tel qu'un prélèvement de surface, d'eau, d'air, .... Cet échantillon peut être ainsi liquide ou solide et on peut citer d'une manière non limitative, un échantillon clinique de sang, de plasma, d'urines, de fécès, de prélèvements de nez, de gorges, de peaux, de plaies, de liquide céphalo-rachidien, un échantillon alimentaire d'eau, de boissons tels que le lait, un jus de fruits; de yaourt, de viande, d'œufs, de légumes, de mayonnaise, de fromage ; de poisson..., un échantillon alimentaire issu d'une alimentation destinée aux animaux, tel que notamment un échantillon issu de farines animales.

L'invention concerne un milieu de détection et de discrimination de bactéries *Citrobacter* parmi d'autres enterobacteries, comprenant
∘ un substrat d'une activité métabolique spécifique d'un groupe d'entérobactéries
∘ un substrat de beta glucosidase ou de cellobiosidase
∘ un inducteur de la beta glucosidase ou cellobiosidase, ledit inducteur étant un hydrate de carbone constitué d'un hydrate de carbone lié en position β au glucose ou hydrate de carbone avec une sous-unité β-glucoside, en l'occurrence le cellobiose.

Les substrats utilisés pour la détection d'une activité beta-glucosidase peuvent notamment être le 4-Méthylumbelliféryl-beta-Glucoside, le 5-Bromo-4-chloro-3-indolyl-beta-Glucoside, le 5-Bromo-6-chloro-3-indolyl-beta-Glucoside, le 6-Chloro-3-indolyl-beta-Glucoside, l'Alizarine-beta-Glucoside, le Cyclohexenoesculetine-beta-Glucoside, le Nitrophényl-beta-glucoside, le Dichloroaminophényl-glucoside ou leurs sels à des concentrations comprises préférentiellement entre 10 et 1000mg/l.

A titre de substrats de cellobiosidase, on peut citer notamment le 4-Méthylumbelliféryl-beta-cellobioside, le 5-Bromo-4-chloro-3-indolyl-beta-cellobioside, le 5-Bromo-6-chloro-3-indolyl-beta-cellobioside, le 6-Chloro-3-indolyl-beta-cellobioside, le Nitrophényl-beta-cellobioside.

Selon un mode préféré de réalisation de l'invention, ledit inducteur de la beta glucosidase ou cellobiosidase est à une concentration entre 10mg/l et 3 g/l.

Selon un mode préféré, les entérobactéries sont des *E. coli.* Ainsi, ledit groupe d'entérobactéries peut comprendre ou être constitué de *E. coli.* Dans ce mode de réalisation, ledit substrat de l'activité métabolique est spécifique d'*E*. *colï* et est préférentiellement choisi parmi un substrat de beta-glucuronidase, beta-galactosidase, alpha-galactosidase, d'acidification du Lactose, de tryptophanase, beta-ribosidase. phosphatase, L-Alanine-aminopeptidase, L-Leucine-aminopeptidase. Préférentiellement ledit substrat est un substrat de beta-glucuronidase ou beta-galactosidase.

Selon un autre mode préféré, les entérobactéries sont des *Salmonella.* Ainsi, ledit groupe d'entérobactéries peut comprendre ou être constitué de *Salmonella.* Dans ce mode de réalisation, ledit substrat de l'activité métabolique est spécifique de *Salmonella* et est préférentiellement choisi parmi un substrat d'alpha galactosidase, estérase, acidification du glucuronate, sorbitol, propanediol, mélibiose, mannitol.

Selon un autre mode préféré de réalisation de l'invention, ledit groupe de bactéries à Gram négatif comprend, ou est constitué de, *Pseudomonas aeruginosa.* Préférentiellement, ledit substrat de l'activité métabolique est spécifique de *Pseudomonas aeruginosa* et est choisi parmi un substrat d'estérase, aminopeptidase, oxydase.

Selon un mode préféré de réalisation de l'invention, le milieu de détection comprend en outre un inducteur de ladite activité métabolique spécifique du groupe de bactéries à Gram négatif, ledit groupe de bactéries à Gram négatif comprenant préférentiellement des entérobactéries.

Selon un mode particulier de réalisation de l'invention, l'inducteur pour la beta-glucuronidase est préférentiellement choisi parmi le glucuronate, le Méthyl-beta-glucuronide ou autres beta-glucuronides.

Selon un autre mode particulier de réalisation de l'invention, l'inducteur pour la beta-galactosidase est préférentiellement choisi parmi le Lactose, l'Isopropyl-beta-thio-galactoside ou autres beta-galactoside.

Selon un mode préféré de réalisation de l'invention, le milieu de détection comprend en outre, un deuxième inducteur de la β-glucosidase ou la cellobiosidase, tel que le Méthyl-β-glucoside, l'Isopropyl-β-thio-glucoside, l'Indoxyl-β-glucoside ou le Méthyl-β-thio-glucoside. Préférentiellement, ledit inducteur est le Méthyl-beta-glucoside.

Préférentiellement, ledit deuxième inducteur est à une concentration comprise entre 100ng/l et 10g/l, préférentiellement entre 10mg/l et 3 g/l, et encore plus préférentiellement entre 50 et 200mg/l.

L'invention concerne également l'utilisation d'un milieu tel que défini ci avant pour discriminer un groupe de bactéries comprenant *Citrobacter* d'un autre groupe de bactéries à Gram négatif. Selon un mode préféré de réalisation de l'invention, le milieu est utilisé pour discriminer un groupe comprenant, ou constitué de *Citrobacter* d'un groupe comprenant, ou constitué de *E. coli.*

Selon un autre mode de réalisation de l'invention, le milieu est utilisé pour discriminer un groupe comprenant, ou constitué de *Citrobacter* d'un groupe comprenant, ou constitué de *Salmonella.*

Selon un autre mode de réalisation de l'invention, le milieu est utilisé pour discriminer un groupe comprenant, ou constitué de *Citrobacter* d'un groupe comprenant, ou constitué de *P. aeruginosa.*

L'invention concerne également un milieu de culture comprenant
∘ un substrat d'une activité métabolique spécifique d'un groupe d'entérobactéries
∘ un substrat de beta glucosidase
∘ un inducteur de la beta glucosidase ou cellobiosidase, ledit inducteur étant un hydrate de carbone constitué d'un hydrate de carbone lié en position β au glucose ou hydrate de carbone avec une sous-unité β-glucoside, en l'occurrence le cellobiose.

Ce milieu est particulièrement adapté pour discriminer un groupe comprenant, ou constitué de des bactéries *Citrobacter* et d'un autre groupe de bactéries à Gram négatif. A ce titre l'invention concerne également l'utilisation d'un milieu tel que défini ci avant pour discriminer un groupe de bactéries comprenant *Citrobacter* d'un autre groupe d'entérobactéries.

Préférentiellement, l'invention concerne l'utilisation d'un milieu tel que défini ci avant pour discriminer
- un groupe comprenant, ou constitué de *Citrobacter* d'un groupe comprenant, ou constitué de *E. coli.*
- un groupe comprenant, ou constitué de *Citrobacter* d'un groupe comprenant, ou constitué de *Salmonella.*
- un groupe comprenant, ou constitué de *Citrobacter* d'un groupe comprenant, ou constitué de *P*. *aeruginosa.*

L'invention concerne également l'utilisation d'un milieu comprenant
∘ un substrat d'une activité métabolique spécifique d'un groupe d'entérobactéries
∘ un substrat de beta glucosidase
∘ un inducteur de la beta glucosidase ou cellobiosidase, ledit inducteur étant un hydrate de carbone constitué d'un hydrate de carbone lié en position β au glucose ou hydrate de carbone avec une sous-unité β-glucoside, en l'occurrence le cellobiose pour discriminer un groupe de bactéries comprenant des *Citrobacter* d'un autre groupe d'enterobactéries, préférentiellement pour discriminer

- un groupe comprenant, ou constitué de *Citrobacter* d'un groupe comprenant, ou constitué de *E. coli.*
- un groupe comprenant, ou constitué de *Citrobacter* d'un groupe comprenant, ou constitué de *Salmonella.*
- un groupe comprenant ou constitué de *Citrobacter* d'un groupe comprenant, ou constitué de *P. aeruginosa.*

L'invention concerne également un procédé pour discriminer un groupe de bactéries comprenant les bactéries *Citrobacter* d'un autre groupe d'enterobactéries, dans un échantillon biologique selon lequel
- on ensemence l'échantillon biologique sur un milieu de détection tel que défini ci avant pour obtenir des colonies de bactéries ou sur un milieu comprenant un substrat d'une activité métabolique spécifique d'un groupe d'entérobactéries un substrat de beta glucosidase, un inducteur de la beta glucosidase ou cellobiosidase, ledit inducteur étant un hydrate de carbone constitué d'un hydrate de carbone lié en position β au glucose ou hydrate de carbone avec une sous-unité β-glucoside, en l'occurrence le cellobiose, pour obtenir des colonies de bactéries
- on identifie les colonies réagissant avec ledit substrat de beta-glucosidase comme appartenant à un groupe comprenant ou constitué de *Citrobacter* et les colonies réagissant seulement avec ledit substrat spécifique d'un groupe d'entérobactéries comme étant des bactéries dudit autre groupe d'enterobactéries.

Préférentiellement, le procédé est utilisé pour discriminer
- un groupe comprenant, ou constitué de *Citrobacter* d'un groupe comprenant, ou constitué de *E. coli.*
- un groupe comprenant, ou constitué de *Citrobacter* d'un groupe comprenant, ou constitué de *Salmonella.*
- un groupe comprenant, ou constitué de *Citrobacter* d'un groupe comprenant, ou constitué de *P. aeruginosa.*

L'ensemencement des microorganismes peut être réalisé par toutes les techniques d'ensemencement connues de l'homme du métier. Une étape d'incubation peut être réalisée à une température pour laquelle l'activité enzymatique que l'on souhaite détecter est optimale, que l'homme du métier peut choisir aisément selon l'activité enzymatique à détecter.

L'identification peut s'effectuer par un examen visuel, par colorimétrie ou fluorimétrie.

### EXEMPLES

Les exemples ci dessous sont donnés à titre explicatif et n'ont aucun caractère limitatif. Ils permettront de mieux comprendre l'invention.

### Exemple 1 : Apport du Cellobiose pour la discrimination entre Escherichia coli et Citrobacter

Différentes concentrations de Cellobiose (0-100-350-1000mg/l) sont ajoutées au milieu CPS ID 3 (bioMérieux). Ces milieux comprennent du 6-Chloro-3-indolyl-beta-glucuronide à 250mg/l et du 5-Bromo-4-chloro-3-indolyl-beta-glucoside à 50mg/l. Ces milieux sont répartis à raison de 20ml par boîte de Petri. Des micro-organismes issus de la collection de la demanderesse ont été ensemencés sur ces milieux par isolement semi-quantitatif de 10µl d'une suspension à 0,5 McFarland diluée au 20^{e}. Les boîtes ont été incubées à 37°C pendant 48 heures. Les colonies formées ont été examinées visuellement après 24 et 48 heures d'incubation. La coloration de ces colonies a été notée. Les résultats sont présentés dans le tableau 1 ci-dessous :

**Tableau 1 : Impact de la concentration en Cellobiose dans le milieu CPS ID 3 sur la coloration des colonies**

| | | Concentration en Cellobiose | | | |
|---|---|---|---|---|---|
| Souches | | 0 | 100 | 350 | 1000 |
| *Escherichia coli* | 24 h | Rose | Rose | Rose | Rose |
| 407 | 48 h | Rouge | Rouge | Rouge | Rouge |
| *Escherichia coli* | 24 h | Rose | Rose | Rose | Rose |
| 003 | 48 h | Rouge | Rouge | Rouge | Rouge |
| *Klebsiella pneumoniae* | 24 h | Turquoise | Turquoise | Turquoise | Turquoise |
| 111 | 48 h | Turquoise | Turquoise | Turquoise | Turquoise |
| *Serratia marcescens* | 24 h | Turquoise | Turquoise | Turquoise | Turquoise |
| 112 | 48 h | Turquoise | Turquoise | Turquoise | Turquoise |
| *Citrobacter freundii* | 24 h | Rose | Violet | Violet | Violet |
| 022 | 48 h | Mauve | Violet | Violet | Violet |
| *Citrobacter freundii* | 24 h | Incolore | Vert | Vert | Incolore |
| 031 | 48 h | Vert | Vert | Vert | Vert |
| *Citrobacter freundii* | 24 h | Incolore | Vert | Vert | Vert |
| 104 | 48 h | Vert | Vert | Vert | Vert |
| *Enterococcus faecalis* | 24 h | Turquoise | Turquoise | Turquoise | Turquoise |
| 117 | 48 h | Turquoise | Turquoise | Turquoise | Turquoise |

Dans le tableau 1 ci-dessus, il apparaît qu'en présence de Cellobiose, les souches de *Citrobacter* expriment une activité beta-glucosidase, ce qui se traduit par une coloration verte à violette des colonies, suivant que la souche exprime ou non parallèlement une activité beta-glucuronidase. Ainsi, en l'absence de Cellobiose, la souche *C. freundii* 022 (colonies roses à 24H) peut être confondue avec les souches d'*Escherichia coli*, ce qui n'est plus le cas en présence de Cellobiose.

### Exemple 2 : Impact de la concentration en Cellobiose sur la discrimination entre Citrobacter et d'autres entérobactéries

Différentes concentrations de Cellobiose (0-0,1-1-5-10g/l) sont ajoutées au milieu Trypcase Soja (bioMérieux) additionné de 5-Bromo-6-chloro-3-indolyl-beta-galactoside à 50mg/l et de 5-Bromo-4-chloro-3-indolyl-beta-glucoside à 50mg/l. Ces milieux sont répartis à raison de 20ml par boîte de Petri. Des micro-organismes issus de la collection de la demanderesse ont été ensemencés sur ces milieux par isolement semi-quantitatif de 10µl d'une suspension à 0,5 McFarland diluée au 20^{e}. Les boîtes ont été incubées à 37°C pendant 48 heures. Les colonies formées ont été examinées visuellement après 24 et 48 heures d'incubation. La coloration de ces colonies a été notée. Les résultats sont présentés dans le tableau 2 ci-dessous :

**Tableau 2: Impact de la concentration en Cellobiose dans le milieu Trypcase soja additionné de 5-Bromo-6-chloro-3-indolyl-beta-galactoside et de 5-Bromo-4-chloro-3-indolyl-beta-glucoside sur la coloration des colonies**

| | | Concentration en Cellobiose | | | | |
|---|---|---|---|---|---|---|
| Souches | | 0 | 0,1 | 1 | 5 | 10 |
| *Escherichia coli* | 24 h | Mauve | Mauve | Mauve | Mauve | Mauve |
| 018 | 48 h | Mauve | Mauve | Mauve | Mauve | Mauve |
| *Escherichia coli* | 24 h | Mauve | Mauve | Mauve | Mauve | Mauve |
| 003 | 48 h | Mauve | Mauve | Mauve | Mauve | Mauve |
| *Klebsiella pneumoniae* | 24 h | Vert | Vert | Vert | Turquoise | Turquoise |
| 111 | 48 h | Bleu | Bleu | Bleu | Bleu | Bleu |
| *Serratia marcescens* | 24 h | Vert | Vert | Vert | Vert | Turquoise |
| 112 | 48 h | Bleu | Bleu | Bleu | Bleu | Bleu |
| *Citrobacter freundii* | 24 h | Mauve | Violet | Violet | Rose | Mauve |
| 022 | 48 h | Mauve | Bleu | Bleu | Bleu | Mauve |
| *Citrobacter freundii* | 24 h | Mauve | Violet | Mauve | Mauve | Mauve |
| 031 | 48 h | Mauve | Vert | Vert | Vert | Mauve |
| *Citrobacter freundii* | 24 h | Mauve | Violet | Violet | Mauve | Mauve |
| 104 | 48 h | Mauve | Vert | Vert | Vert | Mauve |
| *Enterococcus faecalis* | 24 h | Turquoise | Turquoise | Turquoise | Turquoise | Turquoise |
| 117 | 48 h | Turquoise | Turquoise | Turquoise | Turquoise | Turquoise |

Dans le tableau 2 ci-dessus, il apparaît qu'en présence de Cellobiose, les souches de *Citrobacter* expriment une activité beta-glucosidase, ce qui se traduit par une coloration verte à violette des colonies, suivant que la souche exprime ou non parallèlement une activité beta-galactosidase. Ainsi, en l'absence de Cellobiose, les souches *C. freundii* (colonies mauves à 24H) peuvent être confondues avec les souches d'*E. coli,* ce qui n'est plus le cas en présence de Cellobiose. Néanmoins, la différenciation est moins marquée pour des concentrations de Cellobiose supérieures à 5g/l.

## Revendications

1. Milieu de détection et de discrimination de bactéries *Citrobacter* parmi d'autres entérobactéries, comprenant :
∘ un substrat d'une activité métabolique spécifique d'un groupe d'entérobactéries
∘ un substrat chromogène de beta-glucosidase ou de cellobiosidase ;
∘ un inducteur de beta-glucosidase ou cellobiosidase, ledit inducteur étant un hydrate de carbone constitué d'un hydrate de carbone lié en position β au glucose ou hydrate de carbone avec une sous-unité β-glucoside, en l'occurrence le cellobiose.

2. Milieu selon la revendication 1 selon lequel ledit inducteur de la beta-glucosidase ou cellobiosidase est à une concentration comprise entre 10 mg/l et 3 g/l.

3. Milieu selon la revendication 1 ou 2 selon lequel lesdites autres entérobactéries sont des *E. coli.*

4. Milieu selon la revendication 3 selon lequel ledit substrat de l'activité métabolique est spécifique d'*E*. *coli* et est choisi parmi un substrat de beta-glucuronidase, beta-galactosidase, alpha-galactosidase, d'acidification du Lactose, de tryptophanase. beta-ribosidase, phosphatase, L-Alanine-aminopeptidase, L-Leucine-aminopeptidase, préférentiellement un substrat de beta-glucuronidase ou beta-galactosidase.

5. Milieu selon la revendication 1 ou 2 selon lequel lesdites autres entérobactéries sont des *Salmonella.*

6. Milieu selon la revendication 5 selon lequel ledit substrat de l'activité métabolique est spécifique de *Salmonella* et est choisi parmi un substrat d'alpha galactosidase. estérase, acidification du glucuronate, sorbitol, propanediol, mélibiose, mannitol.

7. Milieu selon l'une quelconque des revendications 1 à 6 selon lequel le milieu de détection comprend en outre, un deuxième inducteur de la β-glucosidase ou la cellobiosidase, tel que le Méthyl-β-glucoside, l'Isopropyl-β-thio-glucoside, l'Indoxyl-β-glucoside ou le Méthyl-β-thio-clucoside.

8. Utilisation d'un milieu tel que défini selon l'une quelconque des revendications 1 à 7 pour discriminer un groupe de bactéries comprenant des *Citrobacter* d'un autre groupe d'entérobactéries.

9. Utilisation d'un milieu comprenant
∘ un substrat d'une activité métabolique spécifique d'un groupe d'entérobactéries
∘ un substrat chromogène de beta-glucosidase
∘ un inducteur de la beta-glucosidase ou cellobiosidase, ledit inducteur étant un hydrate de carbone constitué d'un hydrate de carbone lié en position β au glucose ou hydrate de carbone avec une sous-unité β-glucoside, en l'occurrence le cellobiose
∘ pour discriminer un groupe de bactéries comprenant *Citrobacter* d'un autre groupe d'entérobactéries.

10. Procédé pour discriminer un groupe de bactéries comprenant *Citrobacter* d'un autre groupe d'entérobactéries, dans un échantillon biologique selon lequel
1. on ensemence l'échantillon biologique sur un milieu de détection tel que défini dans l'une quelconque des revendications 1 à 7 ou sur un milieu comprenant
• un substrat d'une activité métabolique spécifique d'un groupe d'entérobactéries
• un substrat chromogène de beta-glucosidase
• un inducteur de la beta-glucosidase ou cellobiosidase, ledit inducteur étant un hydrate de carbone constitué d'un hydrate de carbone lié en position β au glucose ou hydrate de carbone avec une sous-unité β-glucoside, en l'occurrence le cellobiose
pour obtenir des colonies de bactéries
2. on identifie les colonies réagissant avec ledit substrat de beta-glucosidase comme appartenant à un groupe comprenant ou constitué de *Citrobacter* et les colonies réagissant seulement avec ledit substrat spécifique d'un groupe d'entérobactéries comme étant des bactéries dudit autre groupe d'entérobactéries.

## Patentansprüche

1. Medium zum Nachweis von *Citrobacter*-Bakterien und zum Unterscheiden derselben von anderen Enterobakterien, das Folgendes umfasst:
o ein Substrat mit einer metabolischen Aktivität, die spezifisch für eine Gruppe von Enterobakterien ist
∘ ein chromogenes Substrat von beta-Glucosidase oder von Cellobiosidase;
∘ einen Induktor von beta-Glucosidase oder Cellobiosidase, wobei es sich bei dem Induktor um ein Kohlenhydrat handelt, das aus einem Kohlenhydrat, welches an der β-Position an Glucose gebunden ist, oder einem Kohlenhydrat mit einer β-Glucosid-Untereinheit besteht, im vorliegenden Fall um Cellobiose.

2. Medium nach Anspruch 1, gemäß welchem der Induktor der beta-Glucosidase oder Cellobiosidase in einer Konzentration vorliegt, die im Bereich von 10mg/L bis 3 g/L liegt.

3. Medium nach Anspruch 1 oder 2, gemäß welchem es sich bei den anderen Enterobakterien um *E*. *coli* handelt.

4. Medium nach Anspruch 3, gemäß welchem das Substrat der metabolischen Aktivität spezifisch für *E*. *coli* ist und aus einem Substrat von beta-Glucuronidase, beta-Galactosidase, alpha-Galactosidase, zur Umwandlung von Lactose in Säure, von Tryptophanase, beta-Ribosidase, Phosphatase, L-Alanin-Aminopeptidase, L-Leucine-Aminopeptidase ausgewählt ist, wobei es sich vorzugsweise um ein Substrat von beta-Glucuronidase oder beta-Galactosidase handelt.

5. Medium nach Anspruch 1 oder 2, gemäß welchem es sich bei den anderen Enterobakterien um *Salmonella* handelt.

6. Medium nach Anspruch 5, gemäß welchem das Substrat der metabolischen Aktivität spezifisch für *Salmonella* ist und aus einem Substrat von alpha-Galactosidase, Esterase, zur Umwandlung von Glucuronat in Säure, Sorbit, Propandiol, Melibiose, Mannit ausgewählt ist.

7. Medium nach einem beliebigen der Ansprüche 1 bis 6, gemäß welchem das Medium zum Nachweis darüber hinaus einen zweiten Induktor der β-Glucosidase oder der Cellobiosidase umfasst, wie etwa Methyl-β-glucosid, Isopropyl-β-thioglucosid, Indoxyl-β-glucosid oder Methyl-β-thioglucosid.

8. Verwendung eines Mediums gemäß der Begriffsbestimmung in einem beliebigen der Ansprüche 1 bis 7, um eine Gruppe von Bakterien, die *Citrobacter* umfasst, von einer anderen Gruppe von Enterobakterien zu unterscheiden.

9. Verwendung eines Mediums, das Folgendes umfasst
∘ ein Substrat mit einer metabolischen Aktivität, die spezifisch für eine Gruppe von Enterobakterien ist
∘ ein chromogenes Substrat von beta-Glucosidase
∘ einen Induktor von beta-Glucosidase oder Cellobiosidase, wobei es sich bei dem Induktor um ein Kohlenhydrat handelt, das aus einem Kohlenhydrat, welches an der β-Position an Glucose gebunden ist, oder einem Kohlenhydrat mit einer β-Glucosid-Untereinheit besteht, im vorliegenden Fall um Cellobiose.
∘ um eine Gruppe von Bakterien, die *Citrobacter* umfasst, von einer anderen Gruppe von Enterobakterien zu unterscheiden.

10. Verfahren, um in einer biologischen Probe eine Gruppe von Bakterien, die *Citrobacter* umfasst, von einer anderen Gruppe von Enterobakterien zu unterscheiden, wobei
1. das Medium zum Nachweis gemäß der Begriffsbestimmung in einem beliebigen der Ansprüche 1 bis 7, oder ein Medium, das Folgendes umfasst, mit der biologischen Probe beimpft wird
• ein Substrat mit einer metabolischen Aktivität, die spezifisch für eine Gruppe von Enterobakterien ist
• ein chromogenes Substrat von beta-Glucosidase
• einen Induktor von beta-Glucosidase oder Cellobiosidase, wobei es sich bei dem Induktor um ein Kohlenhydrat handelt, das aus einem Kohlenhydrat, welches an der β-Position an Glucose gebunden ist, oder einem Kohlenhydrat mit einer β-Glucosid-Untereinheit besteht, im vorliegenden Fall um Cellobiose um Bakterienkolonien zu erhalten
2. diejenigen Kolonien, welche mit dem Substrat von beta-Glucosidase reagieren, als einer Gruppe angehörig erkannt werden, die *Citrobacter* umfasst oder daraus besteht, und diejenigen Kolonien, welche lediglich mit dem Substrat reagieren, das spezifisch für eine Gruppe von Enterobakterien ist, als Bakterien der anderen Gruppe von Enterobakterien.

## Claims

1. A medium of detecting and distinguishing *Citrobacter* from other enterobacteria, comprising:
∘ a substrate for a metabolic activity specific for a group of enterobacteria;
∘ a chromogenic beta-glucosidase or cellobiosidase substrate;
∘ a beta-glucosidase or cellobiosidase inducer, said inducer being a carbohydrate constituted of a carbohydrate linked in the β-position to glucose, or a carbohydrate with a β-glucoside subunit, preferably selected from cellobiose, cellulose, starch, cellotriose and trehalose.

2. The medium as claimed in claim 1, according to which said beta-glucosidase or cellobiosidase inducer is at a concentration of between 100 ng/l and 10g/l, preferably between 10 mg/l and 3 g/l.

3. The medium as claimed in claim 1 or 2, according to which said other enterobacteria *are E. coli.*

4. The medium as claimed in claim 3, according to which said substrate for the metabolic activity is specific for *E. coli* and is selected from a substrate for beta-glucuronidase, beta-galactosidase or alpha-galactosidase, for acidification of lactose, or for tryptophanase, beta-ribosidase, phosphatase, L-alanine aminopeptidase or L-leucine aminopeptidase, preferably a substrate for beta-glucuronidase or beta-galactosidase.

5. The medium as claimed in claim 1 or 2, according to which said other enterobacteria are *Salmonella.*

6. The medium as claimed in claim 5, according to which said substrate for the metabolic activity is specific for *Salmonella* and is selected from a substrate for alpha-galactosidase, esterase or acidification of glucuronate, or sorbitol, propanediol, melibiose or mannitol.

7. The medium as claimed in any one of claims 1 to 6, according to which the detection medium also comprises a second β-glucosidase or cellobiosidase inducer, such as methyl-β-glucoside, isopropyl-β-thioglucoside, indoxyl-β-glucoside or methyl-β-thioglucoside.

8. The use of a medium as defined according to any one of claims 1 to 7, for distinguishing between a group of bacteria comprising *Citrobacter* and another group of enterobacteria.

9. The use of a medium comprising:
∘ a chromogenic beta-glucosidase substrate;
∘ a beta-glucosidase or cellobiosidase inducer, said inducer being a carbohydrate constituted of a carbohydrate linked in the β-position to glucose, or a carbohydrate with the β-glucoside subunit, preferably selected from cellobiose, cellulose, starch and cellotriose,
for distinguishing between a group of bacteria comprising *Citrobacter* and another group of enterobacteria.

10. A method for distinguishing between a group of bacteria comprising *Citrobacter* and another group of enterobacteria, in a biological sample, according to which:
1. the biological sample is inoculated on a detection medium as defined in any one of claims 1 to 12, or on a medium comprising:
• a chromogenic beta-glucosidase substrate;
• a beta-glucosidase or cellobiosidase inducer, said inducer being a carbohydrate constituted of a carbohydrate linked in the β-position to glucose, or a carbohydrate with a β-glucoside subunit, preferably selected from cellobiose, cellulose, starch and cellotriose,
so as to obtain bacterial colonies;
2. the colonies that react with said beta-glucosidase substrate are identified as belonging to a group comprising or constituted of *Citrobacter* and the colonies that react with said substrate specific for a group of enterobacteria are identified as being bacteria belonging to said group of enterobacteria.
